# EUROPÄISCHE PATENTANMELDUNG

(11) **EP 2 030 595 A2**
(43) Veröffentlichungstag der Anmeldung: **04.03.2009**
(21) Anmeldenummer: 08014739.0
(22) Anmeldetag: 20.08.2008
(51) Int. Cl.: A61F 2/18

(54) **Längenvariable Gehörknöchelchenprothese**

(30) Priorität: 31.08.2007 DE 102007041539
(71) Anmelder: Heinz Kurz GmbH Medizintechnik, 72144 Dusslingen (DE)
(72) Erfinder: Steinhardt, Uwe, 72145 Hirrlingen (DE); Kurz, Heinz, 72144 Dusslingen (DE)
(74) Vertreter: Kohler Schmid Möbus

(57) **Zusammenfassung**

Eine Gehörknöchelchenprothese (20) mit einem ersten und zweiten Befestigungselement (21 bzw. 22) zur mechanischen Befestigung im Mittelohr sowie einem mit den Befestigungselementen starr verbundenen und diese miteinander verbindenden Verbindungselement (23), welches ein Aufnahmeteil (24) und ein in eine Aufnahmeöffnung desselben einschiebbares Einschiebeteil (25) aufweist, wobei das Verbindungselement in axialer Richtung zwischen dem Aufnahmeteil und dem Einschiebeteil Längenvariabel gestaltet ist, und wobei das Aufnahmeteil und das Einschiebeteil in einer gewünschten relativen koaxialen Einschiebeposition verklemmbar sind, ist dadurch gekennzeichnet, dass die Klemmkraft F_{K} zwischen dem Aufnahmeteil und dem Einschiebeteil im verklemmten Zustand mindestens 10mal, vorzugsweise etwa 100mal größer ist als die maximalen, natürlicherweise im Mittelohr im Bereich der Gehörknöchelchen auftretenden äußeren Kräfte. Damit kann auf einfache Weise eine gewünschte, definierte Länge der Prothese auch schon vor einem Einklemmen zwischen den beiden Befestigungspunkten hergestellt und auch nach Abschluss der Operation, beispielsweise nach dem Durchstecken eines als Piston ausgebildeten zweiten Befestigungselements durch eine perforierte Steigbügel-Fußplatte, dauerhaft exakt beibehalten werden.

## Beschreibung

Die Erfindung betrifft eine Gehörknöchelchenprothese, die mindestens ein Glied der menschlichen Gehörknöchelchenkette ersetzt oder überbrückt, wobei die Gehörknöchelchenprothese an einem Ende ein erstes Befestigungselement zur mechanischen Verbindung mit dem mit dem Trommelfell oder einem Glied der Gehörknöchelchenkette, insbesondere mit dem Ambossfortsatz oder mit dem Hammergriff, und an ihrem anderen Ende ein zweites Befestigungselement zur mechanischen Verbindung mit einem weiteren Glied oder Teilen eines Gliedes der Gehörknöchelchenkette oder direkt mit dem Innenohr sowie ein die beiden Befestigungselemente Schall leitend miteinander verbindendes, längliches Verbindungselement umfasst, welches ein als Aufnahmeteil ausgebildetes erstes Teilstück und ein koaxial zur Längsachse des Verbindungselements in eine Aufnahmeöffnung des Aufnahmeteils einschiebbares Einschiebeteil ausgebildetes zweites Teilstück aufweist, wobei das erste Befestigungselement mit einem Ende und das zweite Befestigungselement mit dem axial entgegen gesetzten anderen Ende des Verbindungselements mechanisch starr verbunden ist, wobei das Verbindungselement in axialer Richtung zwischen dem Aufnahmeteil und dem Einschiebeteil Längenvariabel gestaltet ist, und wobei die Festlegung der konkreten axialen Länge des Verbindungselements einer individuellen Gehörknöchelchenprothese durch Verklemmen des Einschiebeteiles mit dem Aufnahmeteil in einer gewünschten relativen koaxialen Einschiebeposition erfolgt.

Eine derartige Vorrichtung ist bekannt aus der US 2003/0097178 A1. Das menschliche Mittelohr mit seinen Gehörknöchelchen hat die Aufgabe, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die Otosklerose ist eine Erkrankung des menschlichen Felsenbeins (= Knochen, in dem das gesamte Ohr sitzt), bei der es durch entzündungsähnliche Knochenumbauprozesse zu einer Fixierung des normalerweise locker schwingenden Steigbügels kommen kann. Dadurch wird das Schallsignal nicht oder nur unvollkommen über die Gehörknöchelchenkette auf das Innenohr übertragen, was zur Schwerhörigkeit führt.

Gehörknöchelchenprothesen dienen der Verbesserung der Schallübertragung bei unterschiedlichen pathologischen Befunden. Sie werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese beispielsweise mittels einer Kopfplatte am Trommelfell befestigt und das andere Ende der Gehörknöchelchenprothese beispielsweise am Steigbügel der menschlichen Gehörknöchelchenkette befestigt oder direkt ins Innenohr getaucht wird. Vielfach wird mit den bekannten Gehörknöchelchenprothesen die Schallleitung zwischen dem Trommelfell und dem Innenohr nur begrenzt ermöglicht, weil sie die natürlichen anatomischen Ausbildungen der Gehörknöchelchenkette nur sehr eingeschränkt ersetzen können.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden am Amboss fixiert und ragen über einen Stempel (=Piston) ins Innenohr. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Ein Hauptproblem, das bei jeder Rekonstruktion der menschlichen Gehörknöchelchenkette auftaucht, ist die Auswahl der richtigen Prothesenlänge. Anatomisch bedingt, variieren die jeweils erforderlichen Längen in einem Spektrum von mehreren Millimetern. Daher muss beim operativen Einsetzen einer Gehörknöchelchenprothese entweder eine ausreichend große Auswahl von Prothesen unterschiedlicher axialer Länge bereitgehalten werden oder die verwendeten Gehörknöchelchenprothesen müssen während der Operation ausgehend von einer maximalen Ausgangslänge auf die erforderliche axiale Endlänge gebracht werden können.

In der WO 92/18066 A1 ist eine selbstanpassende Gehörknöchelchenprothese beschrieben, die einen komplizierten und in der Herstellung sehr aufwändigen Federmechanismus in der Verbindung zwischen dem ersten und dem zweiten Befestigungselement aufweist, welcher eine ständige Änderung der axialen Länge der Prothese je nach relativer Lage der Befestigungspunkte im Mittelohr bewirkt. Eine reproduzierbar exakte, feste Längeneinstellung de Prothese, die auch nach dem operativen Einsetzen derselben ins Mittelohr erhalten bleibt, ist damit nicht möglich. Zudem erfordert die bekannte Prothese wegen ihres sehr speziellen mechanischen und geometrischen Aufbaus einen erheblichen Platzbedarf im Mittelohr, so dass sie in vielen Fällen aufgrund der individuellen Gegebenheiten beim Patienten gar nicht einsetzbar ist. Außerdem wird Konstruktionsbedingt nach dem Einsetzen ein nicht unerheblicher permanenter Druck zwischen den beiden Befestigungspunkten im Mittelohr aufgebaut, was einer Heilung nach der Operation nicht gerade förderlich ist und auf Dauer häufig zu postoperativen Komplikationen führt.

Eine Gehörknöchelchenprothese mit während der Operation in bestimmten Grenzen variierbarer axialer Länge ist in der DE 39 01 796 A1 beschrieben. Dabei wird die Längenänderung durch ein Verbiegen des als dünner aus Golddraht gefertigten Verbindungselements erreicht, was allerdings einerseits umständlich in der Handhabung, andererseits ziemlich ungenau ist, so dass damit keine exakte Einstellung der gewünschten axialen Länge der Gehörknöchelchenprothese erreicht werden kann. Außerdem ist das Ergebnis bei dieser Technik nicht immer reproduzierbar und es kann sogar vorkommen, dass sich nach dem Verbiegen des Verbindungselements die eingestellte axiale Länge der Gehörknöchelchenprothese durch ein Zurückfedern des Verbindungselements wieder ändert.

Die EP 0 998 884 A2 beschreibt eine Gehörknöchelchenprothese, bei welcher das als länglicher Schaft ausgebildete erste Verbindungselement durch eine Durchgangsbohrung des als Kopfplatte ausgebildeten ersten Befestigungselements hindurch gesteckt wird, bis eine gewünschte Schaftlänge zwischen dem ersten und dem zweiten Befestigungselement erreicht ist. Sodann wird der Schaft in dieser Position durch Verengen der Durchgangsbohrung in der Kopfplatte fixiert und der über die Kopfplatte hinaus überstehende Teil des Schaftes abgelängt. So erhält man auf einfache Weise eine Prothese mit der jeweils gewünschten bzw. erforderlichen, insbesondere nach der Operation exakt gleich bleibenden axialen Länge.

Aus der DE 10 2005 010 705 B3 ist eine Gehörknöchelchenprothese bekannt, bei der eine intraoperative Variabilität der Prothesenlänge dadurch erreicht wird, dass das längliche Verbindungselement in Form einer Kugelkette ausgebildet ist. Diese wird während der Operation mit einer bestimmten Anzahl von Kugeln durch eine Aufnahmeöffnung des ersten Befestigungselements hindurch gesteckt. Danach wird die Kugelkette in der Aufnahmeöffnung des Befestigungselements durch beiderseits der Kugelkette angreifende federnde Stegelemente fixiert und der durch die Aufnahmeöffnung ragende, überstehende Teil der Kugelkette abgezwickt, so dass die Prothese am Ende schließlich genau die gewünschte axiale Länge aufweist. In ähnlicher Weise wird eine Längenvariabilität auch bei einer Gehörknöchelchenprothesen nach der DE 20 2005 015 944 U1 erreicht, wobei hier wiederum eine abzwickbare Kugelkette als Verbindungselement verwendet wird, jedoch die Aufnahme im ersten Befestigungselement anders gestaltet ist.

Eine weitere Gehörknöchelchenprothese mit intraoperativ veränderbarer axialer Länge ist in der US-A 3,710,399 beschrieben. Hier wird ein zweigeteiltes Verbindungselement zwischen den beiden Befestigungselementen verwendet, das zwei parallel verlaufende gerade Drahtstücke umfasst, von denen das eine vom ersten und das andere vom zweiten Befestigungselement wegragt. Die beiden Drahtstücke können entweder mittels Drahtschlingen an ihren Enden mit dem jeweils andern Drahtstück verbunden oder in eine Art Verbindungsmuffe mit zwei parallelen Längsbohrungen für die beiden Drahtstücke gesteckt werden. Im ersteren Fall ist jedoch die Fixierungsposition und damit die relative Lage der beiden Drahtstücke nur sehr ungenau einzustellen, so dass eine exakte und reproduzierbare Längeneinstellung der Prothese nicht möglich ist. Im zweiten Fall kann es nach dem Einstecken der Drahtstücke in die Verbindungsmuffe leicht zu Verkippungen, Verknickungen oder Verschiebungen der relativen Lagen der Drahtstücke zueinander kommen, wodurch ebenfalls eine genaue Einstellung der axialen Prothesenlänge erschwert bzw. unmöglich gemacht wird.

Wiederum eine andere Technik der Längeneinstellung wird bei einer Gehörknöchelchenprothese angewendet, wie sie aus der DE 10 2005 027 215 A1 bekannt ist. Diese Prothese zielt ausschließlich auf die Situation einer Steigbügel-Operation ab, so dass immer ein kolbenförmiges Piston als zweites Befestigungselement vorgesehen ist. In diesem Piston ist ein Aufnahmemechanismus angeordnet, in welche das schaftförmige Verbindungselement in axialer Richtung eingeschoben werden soll. Von dem Verbindungselement radial abgespreizte Blattfedern sollen dann in einer gewünschten relativen Position zwischen Verbindungselement und zweitem Befestigungselement eine Arretierung bewirken. Abgesehen davon, dass eine exakt reproduzierbare Einstellung einer gewünschten axialen Länge der Prothese damit nicht immer garantiert sein dürfte, ist der Anwendungsbereich dieser Gehörknöchelchenprothese lediglich auf Steigbügel-Operationen beschränkt, bei der über das Piston eine unmittelbare Verbindung zum Innenohr hergestellt wird. Soll jedoch als zweites Befestigungsteil beispielsweise eine Glocke, ein Stempel, ein Clip oder ein flacher Schuh für eine Verbindung mit einem anderen Teil der Gehörknöchelchenkette verwendet werden, ist diese bekannte Prothese nicht verwendbar. Wenn man nämlich einen entsprechenden Aufnahmemechanismus im zweiten Befestigungsteil unterbringen will, so funktioniert dies schon aus geometrischen Gründen nur in einem Kolben, aber niemals in einer Glocke, einem flachen Schuh oder gar in einem Clip.

Eine weitere Gehörknöchelchenprothese ist in der US-A 5,554,188 beschrieben und umfasst wiederum ein als zweigeteilter Schaft aufgebautes Verbindungselement, bei dem der erste, stangenförmige Abschnitt in eine Aufnahmebohrung des als Aufnahmeteil ausgebildeten zweiten Abschnitts einführbar und axial in der Bohrung verschiebbar ist. Um eine gewünschte axiale Länge der Prothese zu erhalten, wird der stangenförmige erste Abschnitt ausgehend von einer maximalen Ausgangslänge auf eine geeignete Endlänge abgeschnitten und bis auf Anschlag in den zweiten Abschnitt eingeschoben. Durch eine entsprechende Gestaltung des lichten Durchmessers der Aufnahmebohrung relativ zum Außendurchmesser des ersten Abschnitts soll nun eine friktionale Verklemmung von erstem und zweitem Abschnitt eine gewisse Fixierung der Prothesenlänge bewirken, wobei die eigentliche Fixierung dadurch erreicht wird, dass sich die gegeneinander beweglichen Teile der Prothese nach der operativen Einführung ins Mittelohr aufgrund ihres jeweiligen Anschlages an den beiden Befestigungspunkten nicht allzu weit voneinander entfernen können. Eine exakt gleich bleibende Prothesenlänge kann damit allerdings auf Dauer nicht sichergestellt werden.

Die Gehörknöchelchenprothese, wie sie in der eingangs zitierten US 2003/0097178 A1 beschrieben ist, weist einen ähnlichen mechanischen Aufbau wie die oben diskutierte Prothese nach der US-A 5,554,188 auf. Darüber hinaus ist sie aber nicht nur als selbstwirkendes passives Element, sondern auch "teil-aktiv" konzipiert und kann eine photothermische Mikropumpe mit bewegtem Fluid umfassen, mittels derer eine selbsttätige Längenadjustierung der Prothese erreicht werden soll.

Aufgabe der vorliegenden Erfindung ist es demgegenüber, eine gattungsgemäße Gehörknöchelchenprothese der eingangs beschriebenen Art mit möglichst einfachen technischen Mitteln dahin gehend zu verbessern, dass eine gewünschte, definierte Länge der Prothese auch schon vor einem Einklemmen zwischen den beiden Befestigungspunkten hergestellt werden kann, und dass diese Länge auch nach Abschluss der Operation, beispielsweise nach dem Durchstecken eines als Piston ausgebildeten zweiten Befestigungselements durch eine perforierte Steigbügel-Fußplatte, sicher und änderungsfrei beibehalten wird.

Erfindungsgemäß wird diese Aufgabe auf ebenso überraschend einfache wie wirkungsvolle Art und Weise dadurch gelöst, dass eine gattungsgemäße Gehörknöchelchenprothese mit den eingangs beschriebenen Merkmalen hinsichtlich ihres geometrischen Aufbaus und durch die Auswahl ihres Materials derart auszulegen ist, dass die Klemmkraft F_{K} zwischen dem Aufnahmeteil und dem Einschiebeteil im verklemmten Zustand mindestens 10mal, vorzugsweise etwa 100mal größer ist als die maximalen, natürlicherweise im Mittelohr im Bereich der Gehörknöchelchen auftretenden äußeren Kräfte.

Letztere variieren je nach Anatomie und Alter eines Patienten freilich etwas. Die obige Vorschrift, wonach die Klemmkraft F_{K} mindestens 10mal, vorzugsweise etwa 100mal größer sein soll als die maximalen, natürlicherweise im Mittelohr im Bereich der Gehörknöchelchen auftretenden äußeren Kräfte, gibt dem Fachmann auf dem Gebiet der Otologie dennoch eine definierte untere Abschätzung für die Klemmkraft F_{K} zwischen dem Aufnahmeteil und dem Einschiebeteil im verklemmten Zustand:
Wie aus dem Fachartikel HNO 3 · 2000 · 48 : 204-208 hervorgeht, funktioniert das menschliche Mittelohr bei Applikation einer Kraft von 2,5mN noch "normal", während bei Einspannung einer Prothese mit einer Kraft von 15mN ein erheblicher Rückgang der Schwingungsamplitude der Steigbügelfußplatte beobachtet wird. Daraus ergibt sich, dass das menschliche Mittelohr offenbar nur für Betriebsbelastungen unterhalb 15mN "ausgelegt" ist.

In dem Fachartikel Laryngo-Rhino-Otol 2007; 86 : 112-116 wird gezeigt, dass durch Anlegen von Kräften ab 290mN und darüber an die Steigbügelfußplatte Frakturen der Steigbügelfußplatte beobachtet werden. Die maximal zulässigen Kräfte, bei denen gerade noch kein Bruch der Steigbügelfußplatte erfolgte, lagen nach dieser Untersuchung unterhalb 250mN.

Somit ist klar, dass die maximalen, natürlicherweise im Mittelohr im Bereich der Gehörknöchelchenkette auftretenden äußeren Kräfte - unabhängig von Anatomie und Alter eines Patienten - im Bereich von höchstens etwa 15mN, möglicherweise auch ein wenig darüber (vielleicht etwa 25 mN) liegen können, damit das Mittelohr "normal" funktioniert. Auf gar keinen Fall dürfen die Kräfte jedoch die Größenordnung von 250mN erreichen. Ansonsten würde es "im Betrieb" des Mittelohrs häufig zu spontanen Frakturen der Steigbügelfußplatte kommen und das menschliche Mittelohr wäre mithin eine krasse "Fehlkonstruktion" - was aber offensichtlich nicht der Fall ist.

Also bedeutet die erfindungsgemäße Vorschrift "mindestens 10mal, vorzugsweise etwa 100mal größer ist als die maximalen, natürlicherweise im Mittelohr im Bereich der Gehörknöchelchen auftretenden äußeren Kräfte" für den einschlägigen Fachmann klar erkennbar:
"Wähle eine Kraft größer als 250mN, vorzugsweise größer als 2,5N".

Nachträgliche postoperative unerwünschte Längen- und/oder Lageänderungen der Prothese werden durch diese erfindungsgemäß vorgeschriebene Wahl der Klemmkraft F_{K} dauerhaft und sicher vermieden.

Die Einhaltung der oben beschriebenen erfindungsgemäßen Lehre ermöglicht unaufwändig und kostengünstig eine echte Längenvariabilität der Gehörknöchelchenprothese "in situ" bzw. intraoperativ, wobei weder größere Sortimente von Prothesen unterschiedlicher Längen während jeder Operation bereitgehalten werden müssen. Außerdem ist die Einstellung der jeweils gewünschten individuellen Prothesenlänge und damit deren Handhabung besonders einfach. Dadurch werden auf simple Weise die Vorteile der oben beschriebenen bekannten Gehörknöchelchenprothese gemäß der gattungsbildenden US 2003/0097178 A1 genutzt, wobei aber auch die Vorteile der in den oben genannten weiteren Druckschriften beschriebenen längenvariablen Prothesen erhalten bleiben und die gemeinsamen Nachteile vermieden werden.

Zudem ist die erfindungsgemäße Gehörknöchelchenprothese universell bei allen denkbaren Arten von Ankoppelungen im Mittelohrraum einsetzbar und nicht auf eine bestimmte Klasse von Operationen beschränkt, während beispielsweise die Prothese gemäß der oben zitierten DE 10 2005 027 215 A1 ausschließlich in der Situation einer Steigbügel-Operation verwendet werden kann.

Bei einer Klasse von Ausführungsformen der Erfindung ist das Einschiebeteil kontinuierlich auf unterschiedlichen koaxialen Positionen, vorzugsweise längs einer vorbestimmten axialen Strecke auf einer beliebigen relativen koaxialen Position mit dem Aufnahmeteil verklemmbar, so dass sich damit jede gewünschte Prothesenlänge unterhalb der vom Grundmuster der Prothese vorgegebenen Maximallänge individuell genau einstellen lässt.

Eine alternative Klasse von Ausführungsformen zeichnet sich dadurch aus, dass das Einschiebeteil auf wählbaren diskreten relativen koaxialen Positionen längs einer axialen Strecke mit dem Aufnahmeteil verklemmbar ist, was die intraoperative Handhabung der Gehörknöchelchenprothese gegenüber einer kontinuierlichen Verschiebbarkeit erleichtert, aber dafür natürlich nur Einstellungen auf die vom Grundmuster der Prothese vorgegebenen diskreten Längen erlaubt.

Besonders einfach zu handhaben sind Weiterbildungen dieser Klasse von Ausführungsformen, bei denen das Einschiebeteil auf der gewählten relativen koaxialen Klemmposition einrastend mit dem Aufnahmeteil verklemmt werden kann, so dass die Prothese in der gewünschten und eingestellten Länge fixiert bleibt und so zwischen den Befestigungspunkten im Mittelohr positioniert werden kann.

Bei einer weiteren Klasse von Ausführungsformen der erfindungsgemäßen Gehörknöchelchenprothese ist das Einschiebeteil mit dem Aufnahmeteil passiv verklemmbar mittels einer eigenen Federwirkung des Einschiebeteils und/oder des Aufnahmeteils.

Alternativ dazu kann aber auch bei einer weiteren Klasse von Ausführungsformen der Erfindung das Einschiebeteil mit dem Aufnahmeteil mittels einer äußeren Einwirkung auf das Einschiebeteil und/oder auf das Aufnahmeteil aktiv verklemmbar gestaltet sein. In einfachen Weiterbildungen dieser Ausführungsformen kann das Einschiebeteil durch Krafteinwirkung von außen, insbesondere mittels Einwirkung einer Crimpzange, mit dem Aufnahmeteil aktiv verklemmbar sein.

Bei eleganteren, allerdings in der Herstellung dafür auch etwas aufwändigeren Weiterbildungen ist vorgesehen, dass das Einschiebeteil durch Wärmeeintrag auf die Gehörknöchelchenprothese von außen, insbesondere mittels Erwärmung der Gehörknöchelchenprothese auf Körpertemperatur, mit dem Aufnahmeteil aktiv verklemmbar ist.

Ganz besonders vorteilhaft sind Varianten dieser Weiterbildungen, bei denen das Einschiebeteil und/oder das Aufnahmeteil ganz oder teilweise aus einem Material mit Formgedächtnis (=memory effect) insbesondere aus Nitinol hergestellt ist. Die Verwendung derartiger Materialien ist auf dem Gebiet der Gehörknöchelchenprothesen an sich bekannt, erweist sich aber gerade im Zusammenhang mit der vorliegenden Erfindung als besonders wirkungsvoll.

Eine geometrisch und ergonomisch günstige Ausführungsform der erfindungsgemäßen Gehörknöchelchenprothese zeichnet sich dadurch aus, dass das Einschiebeteil eine, vorzugsweise an seinem dem Aufnahmeteil zugewandten axialen Ende angeordnete, Verdickung aufweist.

In der Praxis bewähren sich Weiterbildung dieser Ausführungsform, bei denen die Verdickung eine ellipsoide, insbesondere eine rotationsellipsoide, vorzugsweise eine kugelige Form aufweist. Diese Geometrien bereiten auch fertigungstechnisch keine großen Probleme.

Besonders einfach und kompakt aufgebaut und daher zu bevorzugen sind Ausführungsformen der Erfindung, die sich dadurch auszeichnen, dass das Aufnahmeteil einen in Richtung auf das Einschiebeteil hin offenen, länglichen Hohlraum aufweist, der sich in axialer Richtung des Verbindungselements erstreckt.

Bei einer Klasse von Weiterbildungen dieser Ausführungsformen kann der längliche Hohlraum des Aufnahmeteils in Richtung vom Einschiebeteil weg einseitig geschlossen sein und dadurch einen Endanschlag für das Einschiebeteil aufweisen.

Alternativ können diese Ausführungsformen aber auch dahingehend weitergebildet werden, dass der längliche Hohlraum beidseitig in axialer Richtung des Aufnahmeteils offen ist.

Dabei kann der längliche Hohlraum eine zylindrische, vorzugsweise eine kreiszylindrisch Form, oder bei anderen Varianten der Erfindung eine konische oder pyramidale Form aufweisen und sich vorzugsweise in axialer Richtung vom Einschiebeteil weg verjüngen. Möglich sind aber auch Varianten, bei denen der längliche Hohlraum senkrecht zur Längsachse des Aufnahmeteils eine rechteckige Querschnittsform aufweist, was je nach Materialwahl fertigungstechnische Vorteile haben kann.

Ganz besonders einfach in ihrer Handhabung sind Weiterbildungen, bei denen das Verbindungselement ein Klemmteil umfasst, welches das Aufnahmeteil in einer Klemmposition, die der gewünschten relativen koaxialen Einschiebeposition zwischen Einschiebeteil und Aufnahmeteil entspricht, zumindest teilweise umgibt und in dieser Klemmposition den Innendurchmesser des länglichen Hohlraums im Aufnahmeteil mindestens lokal verengt.

Diese Weiterbildungen können nun, je nach gewünschtem Anwendungsgebiet der erfindungsgemäßen Gehörknöchelchenprothese, mechanisch sehr unterschiedliche Ausprägungen aufweisen: So kann etwa das Klemmteil Klammerartig, Manschettenartig, Hülsenartig, Clipartig, Spangenartig, Klemmringartig oder Gürtelartig aufgebaut sein. Der Grundgedanke der vorliegenden Erfindung ist damit im Bereich der Mittelohr-Prothetik ziemlich universell einsetzbar.

Bei bestimmten Ausgestaltungen der Erfindung kann das Klemmteil koaxial von außen über das Aufnahmeteil geschoben werden.

Vorteilhaft können aber auch dazu alternative Ausgestaltungen sein, bei denen das Klemmteil quer zur Längsachse des Aufnahmeteils auf das Aufnahmeteil aufgebracht werden kann.

Diese Ausgestaltungen wiederum können besonders einfach hergestellt werden, wenn das Klemmteil auf einer Seite parallel zur Längsachse des Aufnahmeteils durchgehend geschlitzt ist.

Bei einer ganz besonders bevorzugten Weiterbildung der oben beschriebenen Ausführungsformen weist das Aufnahmeteil auf mindestens einer Seite ein parallel zu seiner Längsachse verlaufendes längliches Fenster auf, welches bis zum länglichen Hohlraum durchbrochen ist und im eingeführten Zustand des Einschiebeteils im Aufnahmeteil einen Blick auf das Einschiebeteil freilässt und dadurch eine einfache und genaue Einstellung der Gehörknöchelchenprothese auf die gewünschte axiale Länge ermöglicht.

Diese Weiterbildung kann noch dadurch weiter verbessert werden, dass neben dem länglichen Fenster parallel zur Längsachse des Aufnahmeteils eine Messskala angeordnet ist, die vorzugsweise quer zur Längsachse des Aufnahmeteils verlaufende, insbesondere äquidistante Skalenstriche enthält, was eine intraoperative und besonders exakte Einstellbarkeit der gewünschten Prothesenlänge erlaubt.

Eine gegenüber zylindrischen Bauteilen erheblich einfachere Fertigung ermöglichen Ausführungsformen der erfindungsgemäßen Gehörknöchelchenprothese, bei denen das Aufnahmeteil in seinem Querschnitt senkrecht zu seiner Längsachse eine rechteckförmige Außenkontur aufweist.

Ganz besonders fertigungsfreundlich und preisgünstig herzustellen sind Weiterbildungen dieser Ausführungsformen, bei denen das Aufnahmeteil aus Blech gefertigt ist.

Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben. Bei einer steifen Verbindung zwischen Kopfplatte und Schaft kann es zwischen der Kante der Kopfplatte und dem Trommelfell bzw. dem Transplantat zwischen Trommelfell und Kopfplatte zu erhöhten Druckspitzen kommen. Diese können so hoch sein, dass eine Penetration oder Extrusion durch das Trommelfell die Folge wäre. Aus diesem Grund ist es sehr hilfreich, wenn die Prothese eine gewisse post-operative Mobilität aufweist, so dass sich die Kopfplatte postoperativ selbstständig der Position des Trommelfells angleichen kann. Da zudem die anatomischen Gegebenheiten des Ohrs, wie beispielsweise die Lage, die Form und die Größe des Steigbügels, des Ambosses, des Hammers und des Trommelfells variieren, ist es sehr vorteilhaft, wenn Gehörknöchelchenprothesen nicht starr ausgebildet sind, sondern eine gewisse Flexibilität oder Variabilität aufweisen.

In der Regel wird bei der erfindungsgemäßen GehörknöchelchenProthese das Verbindungselement zwischen den beiden Befestigungselementen als länglicher Schaft gestaltet sein, wie dies an sich aus dem Stand der Technik wohlbekannt ist. Um die oben erörterte erhöhte Flexibilität bzw. Variabilität der Prothese zu erreichen - wie beispielsweise ausführlich in der EP 1 181 907 B1 beschrieben - kann bei einer besonders bevorzugten Ausführungsform der Erfindung am oder im länglichen Schaft mindestens ein Kugelgelenk vorgesehen sein. Vorteilhaft im Hinblick auf eine besonders hohe postoperative Beweglichkeit der Prothese sind Weiterbildungen, bei denen der längliche Schaft eine Vielzahl von aneinander angrenzenden weiteren Drehelementen, vorzugsweise eine Kugelgelenkkette, umfasst.

Je nach dem individuellen Defekt, der bei einem Patienten durch den Einsatz der erfindungsgemäßen Gehörknöchelchenprothese behoben oder zumindest in seinen Auswirkungen gelindert werden soll, wird der Aufbau der Prothese entsprechend gestaltet sein. In sämtlichen Ausführungsformen der Erfindung wird das erste Befestigungselement eine zur Anlage am Trommelfell ausgebildete Kopfplatte umfassen. Bei vielen Ausführungsformen kann beispielsweise die Prothese einerseits am Ambossfortsatz oder am Steigbügel befestigt sein oder direkt ins Innenohr getaucht werden. Vorteilhaft ist in diesem Zusammenhang eine Ausgestaltung, bei der die Gehörknöchelchenprothese am Endpunkt des Hammers (= Umbo) oder direkt daneben angeordnet ist, wodurch die größte Hebelwirkung für die mechanische Übertragung des Schalls durch Bewegungen in der künstlichen oder natürlichen Gehörknöchelchenkette erzielt wird.

Eine Klasse von Ausführungsformen der erfindungsgemäßen Gehörknöchelchenprothese zeichnet sich dadurch aus, dass das zweite Befestigungselement als Platte, als Hülse, als Schlinge, als geschlossene Glocke, als einfach oder mehrfach geschlitzte Glocke oder als Clip zur mechanischen Verbindung mit einem weiteren Glied der Gehörknöchelchenkette ausgebildet ist.

Bei Weiterbildungen dieser Ausführungsformen ist die Prothese über die Kopfplatte einerseits am Trommelfell und über das zweite Befestigungselement andererseits am Amboss oder am Steigbügel befestigt.

Alternative Ausgestaltungen können vorsehen, dass die Gehörknöchelchenprothese an ihrem das zweite Befestigungselement tragenden Ende mittels Perforation der Steigbügelfußplatte (=Stapedektomie bzw. Stapedotomie) und/oder mittels Eröffnung der menschlichen Hörschnecke (=Cochleotomie) direkt an das Innenohr angekoppelt ist, insbesondere über einen Kolben.

Neben der postoperativen Positionsverschiebung ergibt sich nach der Implantation von Gehörknöchelchenprothesen auch noch ein weiteres Problem: Das Mittelohr des menschlichen Körpers stellt nämlich ein "halb offenes Lager " dar. Jedes Implantationsmaterial, welches im Rahmen einer Rekonstruktion des Mittelohres und seiner Strukturen in den Körper eingebracht wird, erfährt dadurch eine besondere Beanspruchung, dass eine kontaminierte und infizierte Umgebung vorherrscht, die in der Regel das Material angreift. Da das Ziel der Implantation einer Gehörknöchelchenprothese immer auch eine möglichst lange, komplikationsfreie Verweildauer des Implantats im Mittelohr des Patienten sein muss, kann ein lange andauernder Materialangriff zu Beschädigungen der Prothese und/oder zu einer lokalen Infektion führen. Beide Folgen sind nicht tolerabel. Um eine Schädigung sowohl des Implantationsmaterials als auch des umgebenden Gewebes dauerhaft zu verhindern, ist bei einer weiteren besonders bevorzugten Ausführungsform der Erfindung die Oberfläche der Gehörknöchelchenprothese ganz oder zumindest abschnittsweise mit einer biologisch aktiven Beschichtung, insbesondere einer wachstumshemmenden und/oder einer Wachstumsfördernden und/oder einer antibakteriell wirkenden Beschichtung überzogen.

Die Kopfplatte der erfindungsgemäßen Gehörknöchelchenprothese sollte grundsätzlich eine wachstumsfördernde Beschichtung, ein direkt ins Innenohr führendes, etwa in Form eines Kolbens ausgebildetes zweites Befestigungselement hingegen eine wachstumshemmende Beschichtung aufweisen.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Prothese oder Teile davon, insbesondere eines der Befestigungselemente, aus einem Material mit Formgedächtnis (=memory effect) oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können post-operative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen.

Ein solcher Tuning Effekt kann bei speziellen Ausführungsformen etwa dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Eine weitere Ausführungsform der Erfindung zeichnet sich schließlich dadurch aus, dass die Prothese mit einem aktiven Vibrationsteil eines aktiven, insbesondere implantierbaren Hörgeräts verbunden ist. Damit lassen sich auch weitergehende Gehörschäden durch Einsatz moderner Elektronik in weiten Bereichen beheben oder zumindest in ihren Auswirkungen wesentlich lindern, wobei eine körperliche Verbindung der Prothese mit der Außenwelt aufgrund der oben beschriebenen Beschichtung wiederum keine Probleme durch einen erhöhten Bakterieneintrag in den Bereich des Mittelohres verursacht, wenn die Beschichtung entsprechend antibakteriell ausgestaltet ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1a: eine schematische räumliche Darstellung einer ersten Ausführungsform der erfindungsgemäßen Gehör- knöchelchenprothese mit einem Clip als erstem und einer Glocke als zweitem Befestigungselement;
- Fig. 1b: die Ausführungsform nach Fig. 1a in einer Seitenansicht in radialer Richtung zum länglichen Verbindungselement;
- Fig. 1c: die Ausführungsform nach Fig. 1a in einem vertikalen Längsschnitt durch die Gehörknöchelchenprothese;
- Fign. 2a-c: eine Ausführungsform mit einer Trommelfell-Kopfplatte als erstem und einem Piston als zweitem Befestigungselement sowie mit einem Fenster im Aufnahmeteil und einer Mess- skala längs des Fensters; und
- Fign. 3a-c: eine Ausführungsform mit einem Clip als erstem und einem Piston als zweitem Befestigungselement sowie mit einem im länglichen Verbindungselement integrierten Kugelgelenk.

Die in den Figuren der Zeichnung schematisch dargestellten drei - im Detail unterschiedlich gestalteten - Ausführungsformen der erfindungsgemäßen **Gehörknöchelchenprothese 10; 20; 30** weisen am einen Ende jeweils ein **erstes Befestigungselement 11; 21; 31** auf, welches der mechanischen Verbindung der Prothese mit dem Trommelfell oder einem Glied der Gehörknöchelchenkette dient. Am anderen Ende der Gehörknöchelchenprothese 10; 20; 30 sitzt jeweils ein **zweites Befestigungselement 12; 22; 32** zur mechanischen Verbindung der Prothese mit einem weiteren Glied oder Teilen eines Gliedes der Gehörknöchelchenkette oder zum direkten Eintauchen ins Innenohr. Dazwischen ist ein die beiden Befestigungselemente 11; 21; 31 bzw. 12; 22; 32 Schall leitend miteinander verbindendes **Verbindungselement 13; 23; 33** angeordnet, welches bei allen in der Zeichnung gezeigten Ausführungsformen in Form eines länglichen Schaftes ausgeführt ist.

Das Verbindungselement 13; 23; 33 umfasst jeweils ein als **Aufnahmeteil 14; 24; 34** ausgebildetes erstes Teilstück und ein als koaxial zur Längsachse des Verbindungselements 13; 23; 33 in eine Aufnahmeöffnung des Aufnahmeteils 14; 24; 34 einschiebbares **Einschiebeteil 15; 25; 35** ausgebildetes zweites Teilstück, wobei jeweils das erste Befestigungselement 11; 21; 31 mit einem Ende und das zweite Befestigungselement 12; 22; 32 mit dem axial entgegen gesetzten anderen Ende des Verbindungselements 13; 23; 33 mechanisch starr verbunden ist.

Erfindungsgemäß ist das Verbindungselement 13; 23; 33 in axialer Richtung zwischen dem Aufnahmeteil 14; 24; 34 und dem Einschiebeteil 15; 25; 35 Längenvariabel gestaltet, wobei die Festlegung der axialen Länge des Verbindungselements 13; 23; 33 einer individuellen Gehörknöchelchenprothese 10; 20; 30 durch Verklemmen des Einschiebeteils 15; 25; 35 mit dem Aufnahmeteil 14; 24; 34 in einer gewünschten relativen koaxialen Einschiebeposition erfolgt, und wobei die Klemmkraft F_{K} zwischen dem Aufnahmeteil 14; 24; 34 und dem Einschiebeteil 15; 25; 35 im verklemmten Zustand mindestens 10mal, vorzugsweise etwa 100mal größer ist als die maximalen, natürlicherweise im Mittelohr im Bereich der Gehörknöchelchen auftretenden äußeren Kräfte.

Um diese Verklemmung zu bewirken, weist das Einschiebeteil 15; 25; 35 bei den in der Zeichnung dargestellten Ausführungsformen jeweils eine an seinem dem Aufnahmeteil 14; 24; 34 zugewandten axialen Ende angeordnete **Verdickung 16; 26; 36** auf. Diese kann in einen in Richtung auf das Einschiebeteil 15; 25; 35 hin offenen, länglichen **Hohlraum 17; 27; 37** des Aufnahmeteils 14; 24; 34, der sich in axialer Richtung des Verbindungselements 13; 23; 33 erstreckt, eingeführt werden. Die gewünschte Klemmwirkung wird dadurch erreicht, dass der lichte Innendurchmesser des Hohlraums 17; 27; 37 etwas kleiner gewählt ist als der Außendurchmesser der Verdickung 16; 26; 36. Der längliche Hohlraum 17; 27; 37 des Aufnahmeteils 14; 24; 34 ist bei den gezeigten Ausführungsformen in Richtung vom Einschiebeteil 15; 25; 35 weg einseitig geschlossen und bildet dadurch einen Endanschlag für das Einschiebeteil 15; 25; 35.

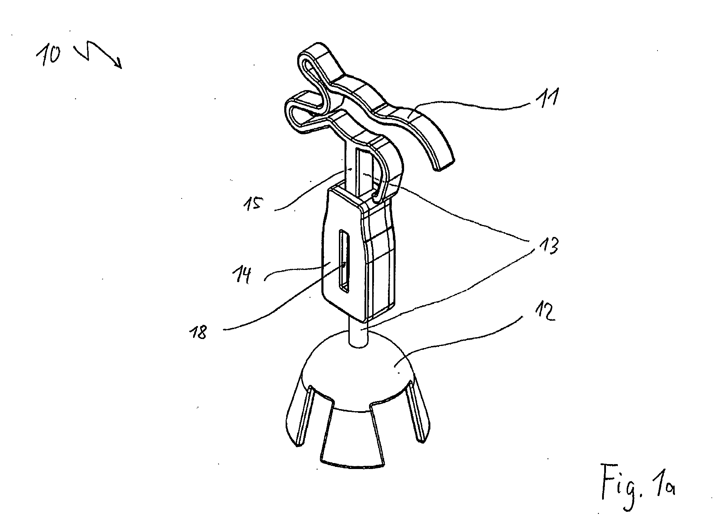

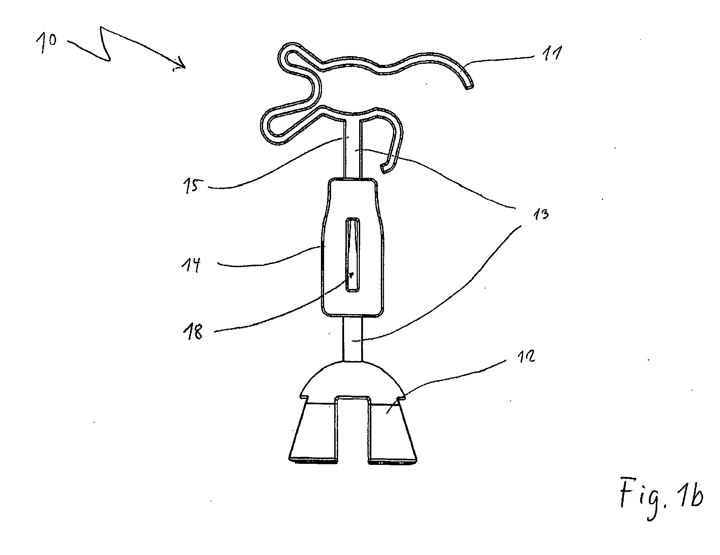

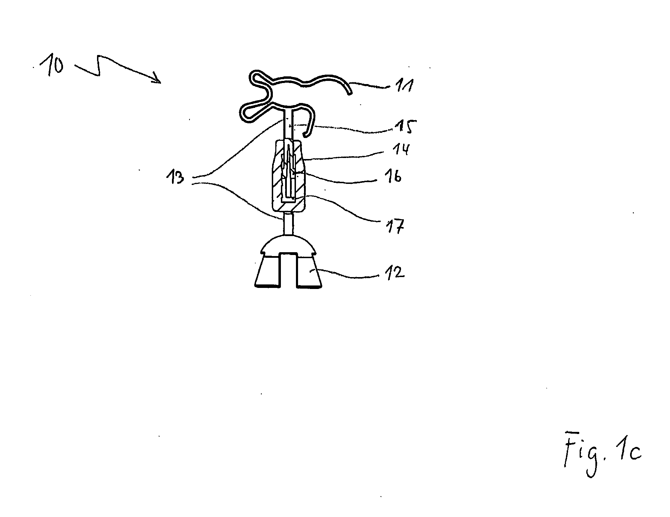

Bei den Ausführungsformen der Figuren 1a - 2c weist das Aufnahmeteil 14; 24 auf mindestens einer Seite ein parallel zu seiner Längsachse verlaufendes längliches **Fenster 18; 28** auf, welches bis zum länglichen Hohlraum 17; 27 durchbrochen ist und im eingeführten Zustand des Einschiebeteils 15; 25 im Aufnahmeteil 14; 24 einen Blick auf das Einschiebeteil 15; 25 freilässt.

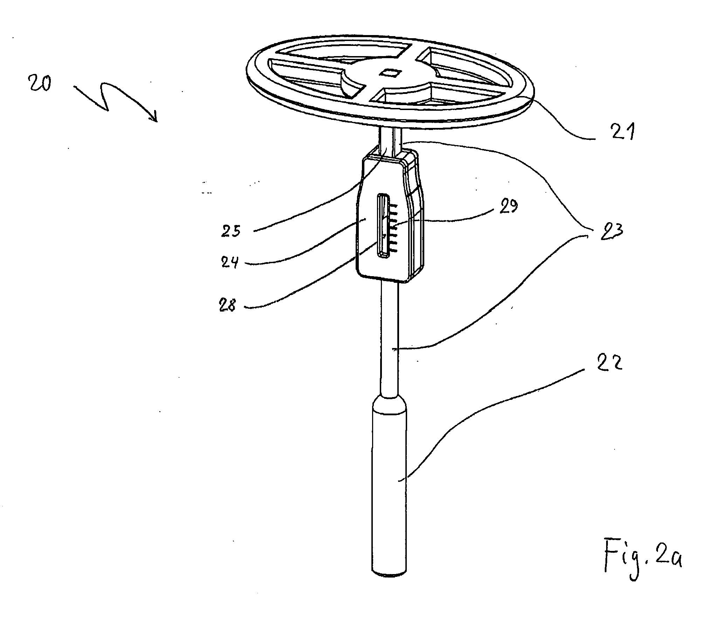

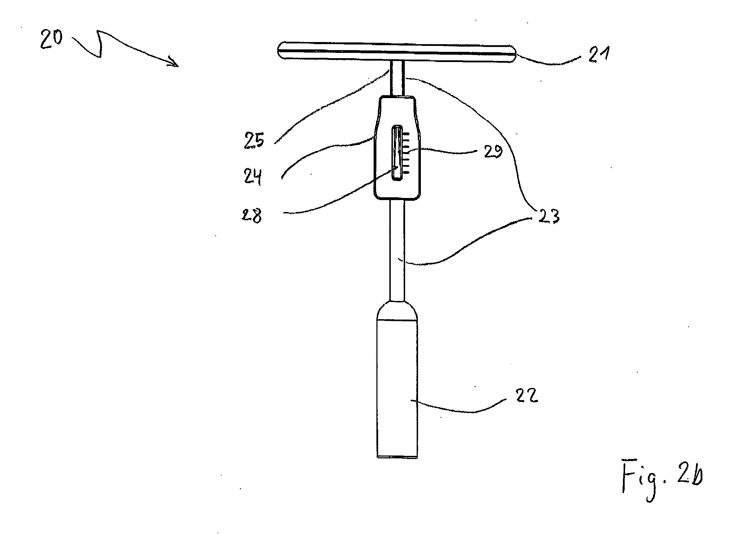

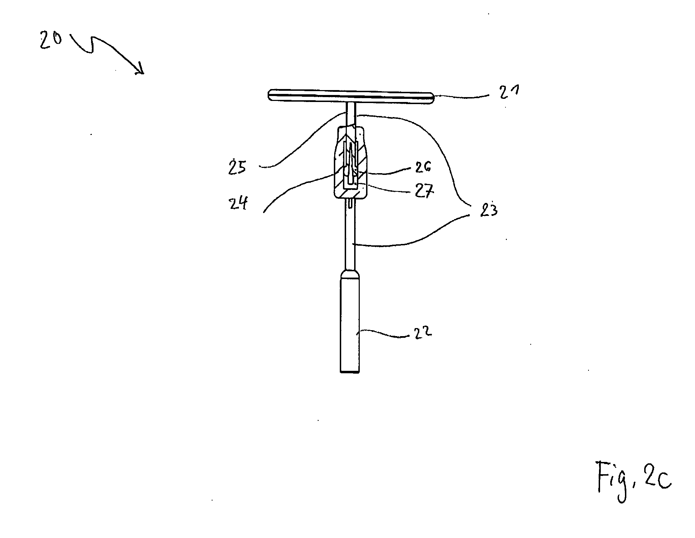

Darüber hinaus ist bei der in den Figuren 2a - 2c dargestellten Ausführungsform neben dem länglichen Fenster 28 parallel zur Längsachse des Aufnahmeteils 24 eine **Messskala 29** angeordnet, die quer zur Längsachse des Aufnahmeteils 24 verlaufende, äquidistante Skalenstriche umfasst.

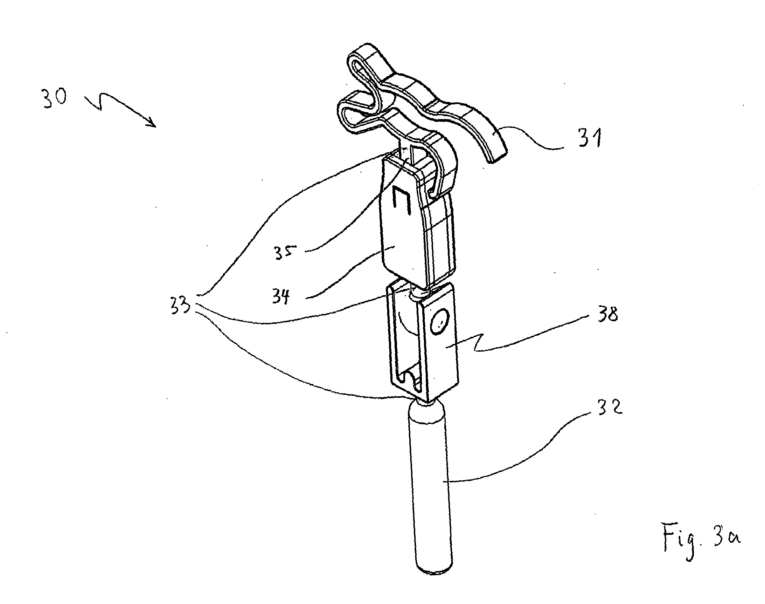

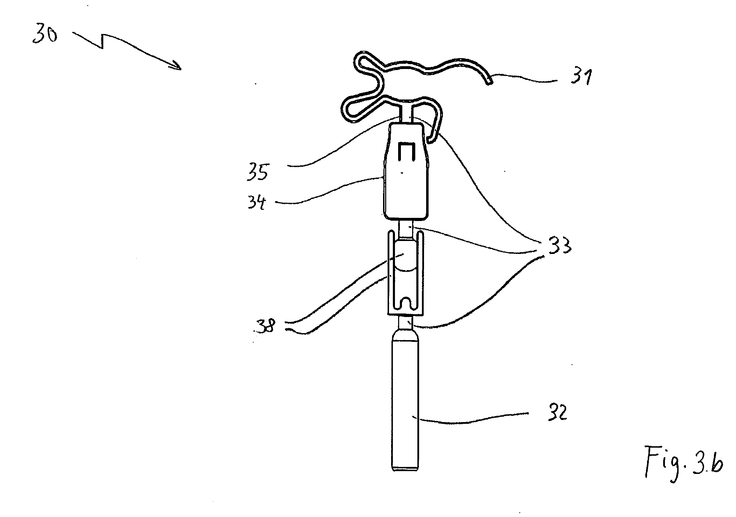

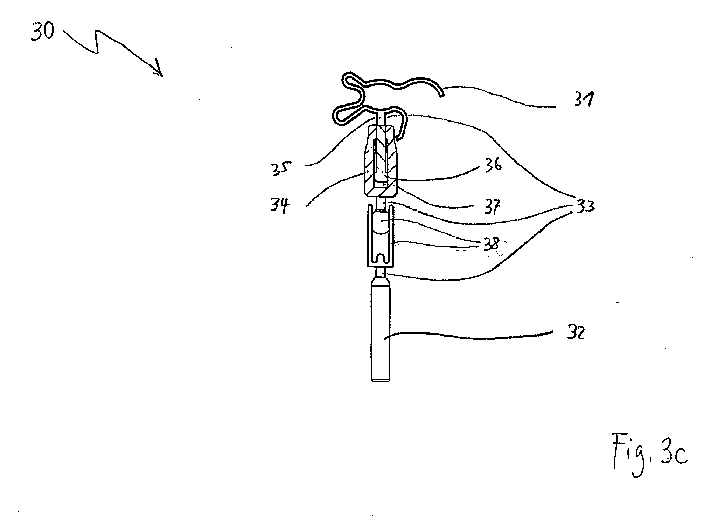

In der Ausführungsform nach den Figuren 3a - 3c ist in das Verbindungselement 33 ein **Kugelgelenk 38** integriert, um eine gewisse postoperative Flexibilität der Gehörknöchelchenprothese 30 zwischen ihren Verbindungsstellen zu erreichen.

Bei der in den Figuren 1a - 1c gezeigten ersten Ausführungsform ist das erste Befestigungselement 11 in Form einer Klammer ausgebildet, die beispielsweise auf den Ambossfortsatz oder auch auf ein anderes Glied der Gehörknöchelchenkette aufgeclipst werden kann. Das zweite Befestigungselement 12 ist hier glockenartig gestaltet und dient vorzugsweise zur Befestigung der Gehörknöchelchenprothese 10 am Steigbügel.

Bei der zweiten Ausführungsform nach Figuren 2a - 2c hingegen ist in das erste Befestigungselement 21 in Form einer Kopfplatte zur Anlage am Trommelfell ausgebildet. Das zweite Befestigungselement 22 an dem der Kopfplatte entgegen gesetzten Ende ist in diesem Ausführungsbeispiel als Kolben zur direkten Ankopplung der Gehörknöchelchenprothese 20 an das Innenohr gestaltet. Letzteres trifft auch für die in den Figuren 3a - 3c dargestellte dritte Ausführungsform einer erfindungsgemäßen Gehörknöchelchenprothese 30 zu, wobei hier allerdings das erste Befestigungselement 31 wieder wie bei der ersten Ausführungsform nach den Figuren 1a - 1c in Form einer Klammer ausgebildet ist.

Die Massenverteilung der einzelnen Teile der erfindungsgemäßen Gehörknöchelchenprothese 10; 20; 30 kann in Abhängigkeit von einem gewünschten, vorgebbaren Frequenzgang der Schallleitung im Mittelohr derart berechnet sein, dass ein individuelles Tuning der SchallleitungsEigenschaften ermöglicht wird.

## Patentansprüche

1. Gehörknöchelchenprothese (10; 20; 30), die mindestens ein Glied der menschlichen Gehörknöchelchenkette ersetzt oder überbrückt, wobei die Gehörknöchelchenprothese (10; 20; 30) an einem Ende ein erstes Befestigungselement (11; 21; 31) zur mechanischen Verbindung mit dem mit dem Trommelfell oder einem Glied der Gehörknöchelchenkette, insbesondere mit dem Ambossfortsatz oder mit dem Hammergriff, und an ihrem anderen Ende ein zweites Befestigungselement (12; 22; 32) zur mechanischen Verbindung mit einem weiteren Glied oder Teilen eines Gliedes der Gehörknöchelchenkette oder direkt mit dem Innenohr sowie ein die beiden Befestigungselemente (11; 21; 31 bzw. 12; 22; 32) Schall leitend miteinander verbindendes, längliches Verbindungselement (13; 23; 33) umfasst, welches ein als Aufnahmeteil (14; 24; 34) ausgebildetes erstes Teilstück und ein als koaxial zur Längsachse des Verbindungselements (13; 23; 33) in eine Aufnahmeöffnung des Aufnahmeteils (14; 24; 34) einschiebbares Einschiebeteil (15; 25; 35) ausgebildetes zweites Teilstück aufweist, wobei das erste Befestigungselement (11; 21; 31) mit einem Ende und das zweite Befestigungselement (12; 22; 32) mit dem axial entgegen gesetzten anderen Ende des Verbindungselements (13; 23; 33) mechanisch starr verbunden ist, wobei das Verbindungselement (13; 23; 33) in axialer Richtung zwischen dem Aufnahmeteil (14; 24; 34) und dem Einschiebeteil (15; 25; 35) Längenvariabel gestaltet ist, und wobei die Festlegung der konkreten axialen Länge des Verbindungselements (13; 23; 33) einer individuellen Gehörknöchelchenprothese (10; 20; 30) durch Verklemmen des Einschiebeteils (15; 25; 35) mit dem Aufnahmeteil (14; 24; 34) in einer gewünschten relativen koaxialen Einschiebeposition erfolgt,
**dadurch gekennzeichnet,**
**dass** die Klemmkraft F_{K} zwischen dem Aufnahmeteil (14; 24; 34) und dem Einschiebeteil (15; 25; 35) im verklemmten Zustand mindestens 10mal, vorzugsweise etwa 100mal größer ist als die maximalen, natürlicherweise im Mittelohr im Bereich der Gehörknöchelchen auftretenden äußeren Kräfte.

2. Gehörknöchelchenprothese nach Anspruch 1, **dadurch gekennzeichnet, dass** das Einschiebeteil (15; 25; 35) auf wählbaren diskreten relativen koaxialen Positionen längs einer axialen Strecke mit dem Aufnahmeteil (14; 24; 34) einrastend verklemmbar ist.

3. Gehörknöchelchenprothese nach einem der Ansprüche 1 oder 2, **dadurch gekennzeichnet, dass** das Einschiebeteil (15; 25; 35) mit dem Aufnahmeteil (14; 24; 34) passiv verklemmbar ist mittels einer eigenen Federwirkung des Einschiebeteils (15; 25; 35) und/oder des Aufnahmeteils (14; 24; 34).

4. Gehörknöchelchenprothese nach einem der vorhergehenden Ansprüche, **dadurch gekennzeichnet, dass** das Einschiebeteil (15; 25; 35) und/oder das Aufnahmeteil (14; 24; 34) ganz oder teilweise aus einem Material mit Formgedächtnis (=memory effect), insbesondere aus Nitinol hergestellt ist.

5. Gehörknöchelchenprothese nach einem der vorhergehenden Ansprüche, **dadurch gekennzeichnet, dass** das Einschiebeteil (15; 25; 35) eine, vorzugsweise an seinem dem Aufnahmeteil (14; 24; 34) zugewandten axialen Ende angeordnete, Verdickung (16; 26; 36) aufweist, die eine ellipsoide, insbesondere eine rotationsellipsoide, vorzugsweise eine kugelige Form aufweist.

6. Gehörknöchelchenprothese nach einem der vorhergehenden Ansprüche, **dadurch gekennzeichnet, dass** das Aufnahmeteil (14; 24; 34) einen in Richtung auf das Einschiebeteil (15; 25; 35) hin offenen, länglichen Hohlraum (17; 27; 37) aufweist, der sich in axialer Richtung des Verbindungselements (13; 23; 33) erstreckt.

7. Gehörknöchelchenprothese nach Anspruch 6, **dadurch gekennzeichnet, dass** der längliche Hohlraum (17; 27; 37) des Aufnahmeteils (14; 24; 34) in Richtung vom Einschiebeteil (15; 25; 35) weg einseitig geschlossen ist und **dadurch** einen Endanschlag für das Einschiebeteil (15; 25; 35) aufweist.

8. Gehörknöchelchenprothese nach einem der Ansprüche 6 oder 7, **dadurch gekennzeichnet, dass** der längliche Hohlraum eine zylindrische, vorzugsweise eine kreiszylindrisch Form aufweist, oder dass der längliche Hohlraum eine konische oder pyramidale Form aufweist und sich vorzugsweise in axialer Richtung vom Einschiebeteil weg verjüngt, oder dass der längliche Hohlraum (17; 27; 37) senkrecht zur Längsachse des Aufnahmeteils (14; 24; 34) eine rechteckige Querschnittsform aufweist.

9. Gehörknöchelchenprothese nach einem der Ansprüche 6 bis 8, **dadurch gekennzeichnet, dass** das Verbindungselement (13; 23; 33) ein Klemmteil umfasst, welches das Aufnahmeteil (14; 24; 34) in einer Klemmposition, die der gewünschten relativen koaxialen Einschiebeposition zwischen Einschiebeteil (15; 25; 35) und Aufnahmeteil (14; 24; 34) entspricht, zumindest teilweise umgibt und in dieser Klemmposition den Innendurchmesser des länglichen Hohlraums (17; 27; 37) im Aufnahmeteil (14; 24; 34) mindestens lokal verengt.

10. Gehörknöchelchenprothese nach Anspruch 9, **dadurch gekennzeichnet, dass** das Klemmteil koaxial von außen über das Aufnahmeteil (14; 24; 34) geschoben werden kann.

11. Gehörknöchelchenprothese nach Anspruch 9, **dadurch gekennzeichnet, dass** das Klemmteil quer zur Längsachse des Aufnahmeteils (14; 24; 34) auf das Aufnahmeteil (14; 24; 34) aufgebracht werden kann.

12. Gehörknöchelchenprothese nach Anspruch 11, **dadurch gekennzeichnet, dass** das Klemmteil auf einer Seite parallel zur Längsachse des Aufnahmeteils (14; 24; 34) durchgehend geschlitzt ist.

13. Gehörknöchelchenprothese nach einem der Ansprüche 6 bis 12, **dadurch gekennzeichnet, dass** das Aufnahmeteil (14; 24) auf mindestens einer Seite ein parallel zu seiner Längsachse verlaufendes längliches Fenster (18; 28) aufweist, welches bis zum länglichen Hohlraum (17; 27) durchbrochen ist und im eingeführten Zustand des Einschiebeteils (15; 25) im Aufnahmeteil (14; 24) einen Blick auf das Einschiebeteil (15; 25) freilässt.

14. Gehörknöchelchenprothese nach Anspruch 13, **dadurch gekennzeichnet, dass** neben dem länglichen Fenster (28) parallel zur Längsachse des Aufnahmeteils (24) eine Messskala (29) angeordnet ist, die vorzugsweise quer zur Längsachse des Aufnahmeteils (24) verlaufende, insbesondere äquidistante Skalenstriche enthält.

15. Gehörknöchelchenprothese nach einem der vorhergehenden Ansprüche, **dadurch gekennzeichnet, dass** das Aufnahmeteil (14; 24; 34) in seinem Querschnitt senkrecht zu seiner Längsachse eine rechteckförmige Außenkontur aufweist.
